(19) Europäisches Patentamt
European Patent Office
Office européen des brevets

(11) Veröffentlichungsnummer: **0 474 063 B1**

(12) # EUROPÄISCHE PATENTSCHRIFT

(45) Veröffentlichungstag der Patentschrift: **01.02.95**

(21) Anmeldenummer: **91114156.2**

(22) Anmeldetag: **23.08.91**

(51) Int. Cl.6: **C07C 309/24**, C07C 49/683, A61K 7/42, A61K 31/125, A61K 31/185

(54) **Dialkoxybenzylidenkampfer-Derivate.**

(30) Priorität: **04.09.90 DE 4027980**

(43) Veröffentlichungstag der Anmeldung: **11.03.92 Patentblatt 92/11**

(45) Bekanntmachung des Hinweises auf die Patenterteilung: **01.02.95 Patentblatt 95/05**

(84) Benannte Vertragsstaaten: **AT BE CH DE ES FR GB IT LI NL SE**

(56) Entgegenhaltungen:
**EP-A- 0 390 682**
**DE-A- 3 833 706**
**GB-A- 2 212 048**
**AT-B- 391 687**
**FR-A- 2 645 145**
**US-A- 4 290 974**

(73) Patentinhaber: **MERCK PATENT GmbH**
**Postfach,**
**Frankfurter Strasse 250**
**D-64271 Darmstadt (DE)**

(72) Erfinder: **Heywang, Ulrich, Dr.**
**Zimmermannweg 7**
**W-6100 Darmstadt (DE)**
Erfinder: **Martin, Roland, Dr.**
**Kastanienweg 21**
**W-6940 Weinheim (DE)**
Erfinder: **Stein, Ingeborg, Dr.**
**Industriestrasse 3,**
**W-6106 Erzhausen (DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.09/3.3.3)

## Beschreibung

Die Erfindung betrifft Dialkoxybenzylidenkampfer-Derivate der Formel I

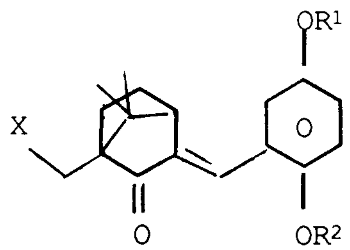

I

worin

$R^1$ und $R^2$ jeweils unabhängig voneinander einen geradkettigen oder verzweigten Alkylrest mit 1 bis 10 C-Atomen, und

X H oder $SO_3H$

bedeuten, sowie Verfahren zu ihrer Herstellung und ihre Verwendung in kosmetischen Zubereitungen, insbesondere zum Schutz vor Sonnenstrahlung, und in pharmazeutischen Zubereitungen zur vorbeugenden Behandlung von Entzündungen und Allergien der Haut oder bestimmter Krebsarten.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten. Im extremen Fall kommt es bei manchen Menschen zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Es ist bekannt, daß die in kosmetischen Präparaten enthaltenen Komponenten nicht immer genügend lichtstabil sind und sich unter der Einwirkung von Lichtstrahlen zersetzen.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Es erscheint somit wünschenswert, Verbindungen zur Verfügung zu stellen, welche UV-Strahlen in einem Wellenlängenbereich von 280 bis 400 nm absorbieren können, d.h. auch UV-B-Strahlen mit einer Wellenlänge zwischen 280 und 320 nm, die bei der Bildung eines Sonnenerythems eine entscheidende Rolle spielen, wie auch UV-A-Strahlen mit einer Wellenlänge zwischen 320 und 400 nm, welche die Haut bräunen aber auch altern lassen, die Auslösung einer erythematösen Reaktion begünstigen oder diese Reaktion bei bestimmten Menschen vergrößern oder sogar phototoxische oder photoallergische Reaktionen auslösen können.

Die heute üblichen Sonnenschutzfilter in der Kosmetik werden in UVA- bzw. UVB-Filter unterteilt. Während es im UVB-Bereich (280-320 nm) mit Substanzen wie Eusolex® 6300 oder Eusolex® 232 gute Filter gibt, sind die im UVA-Bereich (320-400 nm) verwendeten problembehaftet:

Dibenzoylmethane wie Parsol® 1789 oder Eusolex® 8020 sind bei UV-Bestrahlung nicht unbegrenzt stabil, was zum einen die Filtereffektivität mit der Zeit herabsetzt und zum anderen Photosensibilisierungen der Haut in vereinzelten Fällen begünstigen kann. Die ebenfalls als UVA-Filter verwendeten Benzophenone sind in den in der Kosmetik verwandten Ölen nur begrenzt löslich, und sie weisen eine relativ geringe Absorption auf. Dagegen sind nur wenige wasserlösliche UVA-Filter derzeit bekannt, deren UV-Absorption jedoch gering ist.

Aus der DE-OS 38 33 706 sind ähnliche Benzylidenkampfer-Derivate bekannt. Diese weisen jedoch mindestens eine tertiäre Alkylgruppe auf. Diese Verbindungen können zwar auch als UV-Filter in Sonnenschutzmitteln verwendet werden, eignen sich jedoch aufgrund der phenolischen Hydroxylgruppe eher als Antioxidantien. Aufgrund ihrer tertiären Alkylgruppe sind diese Verbindungen in herkömmlichen kosmetischen Trägern, insbesondere in wäßrigen Suspensionen, nur begrenzt löslich, so daß sie in Sonnenschutzmitteln immer zusammen mit anderen UV-Filtern eingesetzt werden müssen.

Es wurde gefunden, daß 3-(2',5'-Dialkoxybenzyliden)kampfer, insbesondere 3-(2',5'-Dimethoxybenzyliden)kampfer, hervorragende UVA-Filter-Eigenschaften besitzt. Ihre Löslichkeit in den in der Kosmetik

verwandten Ölen ist sehr gut, so daß Einsatzkonzentrationen auch in komplizierten Formulierungen bis mindestens 10 % der Zubereitung möglich sind. Eine wasserlösliche Form des neuen Filters sind 3-(2',5'-Dialkoxy-benzyliden)kampfer-10-sulfonsäuren. Die Wasserlöslichkeit ist hier so gut, daß ebenfalls Einsatzkonzentrationen von 10 % möglich sind.

Die Extinktion hat darüber hinaus im UVB-Bereich ein Minimum; dies ist jedoch kein Nachteil, da man problemlos einen UVB-Filter mit in die Formulierung einarbeiten kann.

Weiterhin können die Verbindungen der Formel I auch zur vorbeugenden Behandlung von Entzündungen und Allergien der Haut und zur Verhütung bestimmter Krebsarten verwendet werden.

Außer ihren guten Eigenschaften als Filter zeichnen sich die erfindungsgemäßen Verbindungen durch eine gute thermische und photochemische Stabilität aus.

Ferner bieten diese Verbindungen den Vorteil, nicht toxisch oder reizend und gegenüber der Haut vollkommen unschädlich zu sein.

Sie verteilen sich gleichmäßig in den herkömmlichen kosmetischen Trägern und können insbesondere in Fett-Trägern einen kontinuierlichen Film bilden; sie können auf diese Weise auf die Haut aufgetragen werden, um einen wirksamen Schutzfilm zu bilden.

Gegenstand der Erfindung sind die Verbindungen der oben gegebenen Formel I.

In dieser Formel steht $R^1$ bzw. $R^2$ z.B. für einen Methyl-, Ethyl-, n-Propyl-, i-Propyl-, n-Butyl-, i-Butyl-, t-Butyl- oder 1,1,3,3-Tetramethylbutylrest.

Zu den bevorzugten Verbindungen der allgemeinen Formel I zählen:

3-(2',5'-Dimethoxybenzyliden)kampfer
3-(2',5'-Diethoxybenzyliden)kampfer
3-(2',5'-Dipropoxybenzyliden)kampfer
3-(2',5'-Dimethoxybenzyliden)kampfer-10-sulfonsäure
3-(2',5'-Diethoxybenzyliden)kampfer-10-sulfonsäure
3-(2',5'-Dipropoxybenzyliden)kampfer-10-sulfonsäure

Man erhält die Verbindungen der Formel I z.B. dadurch, daß man einen aromatischen Aldehyd der Formel II,

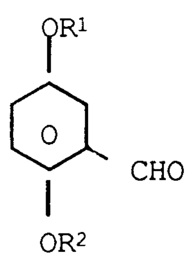

II

worin $R^1$ und $R^2$ die angegebene Bedeutung besitzen, mit natürlichem oder synthetischem Kampfer oder Kampfer-10-sulfonsäure in Gegenwart einer mineralischen Base oder eines Dialkylacyloxyborans kondensiert.

Die Aldehyde der Formel II werden nach bekannten Methoden hergestellt.

Die Kondensation des Aldehyds II mit Kampfer kann vorzugsweise nach einem der zwei folgenden Verfahren durchgeführt werden:

1. Verfahren

Die Kondensation erfolgt in Anwesenheit eines Alkalimetallalkoholates, wie Natriummethylat oder Kalium-t-butylat, in einem Lösungsmittel, wie Toluol, bei Rückflußtemperatur des Lösungsmittels. Die Kondensierung kann auch in Anwesenheit einer mineralischen Base, wie einem Alkalimetallamid oder -hydrid, in Anwesenheit eines Lösungsmittels, wie Dimethoxyethan, und bei Rückflußtemperatur des Lösungsmittels durchgeführt werden.

2. Verfahren

Die Kondensation des Aldehyds II mit Kampfer erfolgt in Anwesenheit eines Borans der folgenden Formel III

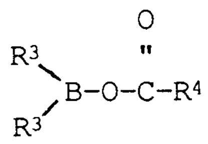

III

worin

$R^3$ für einen Alkylrest mit 1 bis 6 Kohlenstoffatomen steht, und

$R^4$ für einen Alkylrest mit 1 bis 4 Kohlenstoffatomen steht.

Man erhält diese Verbindung nach dem in J. Am. Chem. Soc. 87, 1236 (1965) beschriebenen Verfahren. Es ist nicht notwendig, die Verbindung zu isolieren und zu reinigen, um die Kondensierung des Aldehyds II mit Kampfer durchzuführen.

Die Kondensation wird bei einer Temperatur von 140-160 °C ohne Lösungsmittel durchgeführt.

Gegenstand der Erfindung ist auch das Verfahren zur Herstellung der neuen Verbindungen der Formel I.

Ein weiterer Gegenstand der Erfindung ist eine kosmetische Zubereitung, welche in einem kosmetisch verträglichen Träger eine wirksame Menge mindestens eines Benzylidenkampfer-Derivates der obigen Formel I enthält.

Insbesondere bevorzugt sind solche kosmetischen Zubereitungen, worin der Träger mindestens eine Fettphase aufweist und in der Verbindung der Formel I X H bedeutet, bzw. solche, worin der Träger mindestens eine wäßrige Phase aufweist und X $SO_3H$ bedeutet.

Das erfindungsgemäße kosmetische Mittel kann als Mittel zum Schutz der menschlichen Epidermis oder der Haare oder als Sonnenschutzmittel verwendet werden.

Gegenstand der Erfindung ist ferner ein Verfahren zum Schutz der Haut und natürlicher oder sensibilisierter Haare von Sonnenstrahlen, wobei auf die Haut oder die Haare eine wirksame Menge mindestens einer Verbindung der Formel I aufgetragen wird.

Mit "sensibilisierten Haaren" sind Haare gemeint, welche einer Dauerwellenbehandlung, einem Färbe- oder Entfärbeprozeß unterzogen worden sind.

Gegenstand der Erfindung ist ferner eine gefärbte oder ungefärbte lichtstabilisierte kosmetische Zubereitung, welche eine wirksame Menge mindestens eines Benzyliden~ kampfer-Derivates der obigen Formel I umfaßt.

Wird das erfindungsgemäße kosmetische Mittel als Mittel zum Schutz menschlicher Epidermis gegen UV-Strahlen verwendet, so liegt es in verschiedenen, für diesen Typ üblicherweise verwendeten Formen vor. So kann es insbesondere in Form öliger oder ölig-alkoholischer Lotionen, Emulsionen, wie als Creme oder als Milch in Form ölig-alkoholischer, öligwäßriger oder wäßrig-alkoholischer Gele oder als feste Stifte vorliegen oder als Aerosol konfektioniert sein.

Es kann kosmetische Adjuvanzien enthalten, welche in dieser Art von Mittel üblicherweise verwendet werden, wie z.B. Verdickungsmittel, weichmachende Mittel, Befeuchtungsmittel, grenzflächenaktive Mittel, Konservierungsmittel, Mittel gegen Schaumbildung, Parfums, Wachse, Lanolin, Treibmittel, Farbstoffe und/oder Pigmente, welche das Mittel selbst oder die Haut färben, und andere in der Kosmetik gewöhnlich verwendete Ingredienzien.

Die Verbindung der Formel I ist in der Regel in einer Menge von 1 bis 10 % bezogen auf das Gesamtgewicht des kosmetischen Mittels zum Schutz menschlicher Epidermis, enthalten.

Man kann als Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der Verbindung der Formel I Fettalkohole, Fettsäureester, insbesondere Triglyceride von Fettsäuren, Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Das erfindungsgemäße kosmetische Mittel kann auch als alkoholisches Gel vorliegen, welches einen oder mehrere Niedrigalkohole oder -polyole, wie Ethanol, Propylenglycol oder Glycerin, und ein Verdik-

kungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäureestern, Lanolin und anderen Fettkörpern.

Gegenstand der Erfindung sind auch kosmetische Sonnenschutzmittel, die mindestens eine Verbindung der Formel I enthalten und andere UVB- und/oder UVA-Filter umfassen können.

In diesem Fall beträgt die Menge des Filters der Formel I in der Regel zwischen 1,0 und 8,0 Gew.% bezogen auf das Gesamtgewicht des Sonnenschutzmittels.

Ist ein Mittel als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor UV-Strahlen schützen, so kann es als Shampoo, Lotion, Gel oder Emulsion zum Ausspülen vorliegen, wobei die jeweilige Formulierung vor oder nach dem Shamponieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der erfindungsgemäßen Verbindung verschiedene, in diesem Mitteltyp verwendete Adjuvantien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien. Das Mittel enthält in der Regel 1,0 bis 5,0 Gew.% der Verbindung der Formel I.

Die vorliegende Erfindung befaßt sich auch mit kosmetischen Mitteln, die mindestens eine Verbindung der Formel I als Mittel zum Schutz vor UV-Strahlen und als Antioxidationsmittel enthalten; diese Mittel umfassen Haarprodukte, wie Haarlacke, Wasserwell-Lotionen zum Einlegen der Haare, gegebenenfalls zum Behandeln oder leichteren Frisieren, Shampoos, Färbeshampoos, Haarfärbemittel, Schminkprodukte, wie Nagellack, Cremes und Öle zur Hautbehandlung, Make-up (Fond de teint), Lippenstifte, Hautpflegemittel, wie Badeöle oder -cremes und andere kosmetische Mittel, die im Hinblick auf ihre Komponenten Probleme mit der Lichtstabilität und/oder der Oxidation im Laufe des Lagerns aufwerfen können. Derartige Mittel enthalten in der Regel 1,0 bis 5,0 Gew.% einer Verbindung der Formel I.

Ferner befaßt sich die Erfindung mit einem Verfahren zum Schutz der kosmetischen Mittel vor UV-Strahlen und Oxidation, wobei diesen Mitteln eine wirksame Menge mindestens einer Verbindung der Formel I zugesetzt wird.

Ein weiterer Gegenstand der Erfindung ist die Verwendung der Verbindungen der Formel I als Sonnenfilter von großer Absorptionsbreite in einem Wellenlängenbereich von 320 bis 400 nm.

Ein weiterer Gegenstand der Erfindung ist die Verwendung der Verbindungen der Formel I als kosmetische Produkte.

Wie oben bereits erwähnt, hat die Anmelderin im Laufe ihrer Untersuchungen außerdem festgestellt, daß die Verbindungen der Formel I eine signifikante pharmakologische Aktivität auf dem Gebiet der Präventivbehandlung von Entzündungen und Hautallergien zeigen.

Gegenstand der Erfindung sind auch die Verbindungen der Formel I zur Verwendung als Medikament.

Gegenstand der Erfindung ist ferner ein pharmazeutisches Mittel, welches eine wirksame Menge mindestens einer Verbindung der Formel I als Wirkstoff in einem nicht-toxischen Träger oder Exzipienten enthält.

Das erfindungsgemäße pharmazeutische Mittel kann oral oder topisch verabreicht werden.

Für die orale Verabreichung liegt das pharmazeutische Mittel in Form von Pastillen, Gelatinekapseln, Dragees, als Sirup, Suspension, Lösung, Emulsion etc. vor. Zur topischen Verabreichung liegt es als Salbe, Creme, Pomade, Lösung, Lotion, Gel, Spray, Suspension etc. vor.

Dieses Mittel kann inerte oder pharmakodynamisch aktive Additive enthalten, insbesondere Hydratisierungsmittel, Antibiotika, steroide oder nicht-steroide antiinflammatorische Mittel, Carotinoide und Mittel gegen Psoriasis.

Dieses Mittel kann auch Geschmacksverbesserungsmittel, Konservierungsmittel, Stabilisatoren, Feuchtigkeitsregulatoren, pH-Regulatoren, Modifikatoren für den osmotischen Druck, Emulgatoren, Lokalanaesthetika, Puffer etc. enthalten.

Es kann außerdem in an sich bekannter Weise in Retard-Form oder in einer Form konditioniert sein, in welcher der Wirkstoff rasch freigesetzt wird.

Herstellungsbeispiele

Beispiel 1

DL-3-(2'-5'-Dimethoxybenzyliden)kampfer

In einer mit Stickstoff inertisierten Apparatur wird eine Suspension von 9,2 g (0,17 mol) Natriummethanolat in 200 ml Cyclohexan vorgelegt und mit einer Lösung von 18,2 g (0,12 mol) DL-Kampfer in 100 ml Cyclohexan versetzt. Unter Rühren wird auf 50 °C erhitzt und dann 25 g (0,15 mol) aufgeschmolzener 2,5-Dimethoxybenzaldehyd zugegeben. Das Reaktionsgemisch wird 3 Stunden unter Rückfluß erhitzt. Nach dem Abkühlen wird mit 300 ml Wasser versetzt. Die organische Phase wird abgetrennt, mit 1 n Salzsäure und anschließend mit gesättigter Kochsalzlösung gewaschen und über Natriumsulfat getrocknet. Nach dem Entfernen von Cyclohexan im Vakuum erhält man 17,1 g Rohprodukt. Überschüssiger DL-Kampfer wird durch eine Wasserdampfdestillation entfernt. Nach Chromatographie an Kieselgel 60 mit Toluol/Essigester im Verhältnis 3:1 als Elutionsmittel erhält man ein Öl, welches nach einiger Zeit zu 9,2 g kristallinem 3-(2',5'-Dimethoxybenzyliden)kampfer vom Schmelzpunkt 70-72 °C erstarrt.

Die Substanz zeigt IR-, NMR- und Massenspektren, die der erwarteten Struktur entsprechen.

UV (iso-Propanol) $\lambda\ max_1$ = 287 nm, $\epsilon$ = 12000
$\lambda\ max_2$ = 352 nm, $\epsilon$ = 6500

Analog werden hergestellt:

D-(+)-3-(2',5'-Dimethoxybenzyliden)kampfer
Schmelzpunkt 95-98 °C, $[\alpha]_D^{20}$ = +388,1° (c = 1, Ethanol)
DL-3-(2',5'-Diethoxybenzyliden)kampfer
DL-3-(2',5'-Di-n-propoxybenzyliden)kampfer
DL-3-(2',5'-Diisopropoxybenzyliden)kampfer
DL-3-(2',5'-Di-n-butoxybenzyliden)kampfer
DL-3-(2',5'-Di-tert.butoxy)benzyliden)kampfer
DL-3-(2',5'-Di-n-hexyloxy)benzyliden)kampfer

Beispiel 2

DL-3-(2',5'-Dimethoxybenzyliden)kampfer-10-sulfonsäure

In einer mit Stickstoff inertisierten Apparatur werden 46,5 g (0,2 mol) DL-Kampfer-10-sulfonsäure (wasserfrei) vorgelegt und mit 400 ml Toluol und 20 ml Methanol versetzt. In diese Lösung werden 27,0 g (0,5 mol) Natriummethanolat eingetragen und auf Rückfluß erhitzt. Nach 30 min wird zu der am Rückfluß kochenden Lösung binnen einer Stunde die Lösung von 16,6 g (0,1 mol) 2,5-Dimethoxybenzaldehyd in 400 ml eines 3:1-Gemisches von Toluol und Methanol getropft. Anschließend wird noch weitere 30 h unter Rückfluß erhitzt. Der Ansatz wird mit 150 ml halbkonzentrierter Salzsäure versetzt, bis der pH kleiner als 3 ist. Die wäßrige Phase wird abgetrennt und 2x mit Essigester (jeweils 100 ml) extrahiert. Die vereinigten organischen Phasen werden über $Na_2SO_4$ getrocknet und anschließend im Vakuum (Badtemperatur ca. 40 °C) bis zur einsetzenden Kristallisation eingeengt. Die verbleibende Kristallsuspension wird über Nacht bei -20 °C gehalten und anschließend abgesaugt. Man erhält so 20 g DL-3-(2',5'-Dimethoxybenzyliden)-kampfer-10-sulfonsäure vom Schmelzpunkt 121 bis 123 °C.

Die Substanz zeigt IR-, NMR- und Massenspektren, die der erwarteten Struktur entsprechen.

UV (Methanol): $\lambda\ max_1$ = 258 nm, $\epsilon$ = 8400
$\lambda\ max_2$ = 286 nm, $\epsilon$ = 13400
$\lambda\ max_3$ = 352 nm, $\epsilon$ = 7400

Analog werden hergestellt:

DL-3-(2',5'-Diethoxybenzyliden)kampfer-10-sulfonsäure
DL-3-(2',5'-Di-n-propoxybenzyliden)kampfer-10-sulfonsäure
DL-3-(2',5'-Di-n-butoxybenzyliden)kampfer-10-sulfonsäure
DL-3-(2',5'-Di-n-hexyloxybenzyliden)kampfer-10-sulfonsäure
DL-3-(2',5'-Di-tert.-butyloxybenzyliden)kampfer-10-sulfonsäure

DL-3-(2',5'-Diisopropoxybenzyliden)kampfer-10-sulfonsäure

In den folgenden Anwendungsbeispielen A bis D und H können wahlweise jeweils folgende UV-Filter eingesetzt werden:

1. 10 % Verbindung aus Beispiel 1
2. 5 % Verbindung aus Beispiel 1
   2 % Benzophenon-3
   3 % Octyldimethyl-p-aminobenzoesäureester (Octyldimethylpaba)
3. 6 % Verbindung aus Beispiel 1
   3 % 3-(4-Methylbenzyliden)kampfer
4. 4 % Verbindung aus Beispiel 1
   1 % Octyltriazon
5. 8 % Verbindung aus Beispiel 1
   6 % Octylmethoxycinnamat
6. 6 % Verbindung aus Beispiel 1
   4 % Octyl-Salicylat
   2 % 4-Isopropyldibenzoylmethan
7. 8 % Verbindung aus Beispiel 1 2 % Benzophenon-3

Die in Klammern angegebenen Firmenbezeichnungen geben die Bezugsquelle der jeweiligen Ingredienzien an.

Anwendungsbeispiele

Beispiel A: Sonnenschutzmittel (O/W)

Man mischt folgende Ingredienzien, wobei sie zum Homogenisieren gegebenenfalls auf 70-85 °C erhitzt werden; das Parfumöl wird bei 35-45 °C zugegeben.

| | Gew. % |
|---|---|
| UV-Filter | q.s. |
| Arlacel 165 "Glycerinmonostearat und PDÄ-"Stearat" (ICI, Essen) | 9,5 |
| Atlas G-1790 "Polyoxyethylen(20)lanolin-Derivat" (ICI, Essen) | 6,60 |
| Lanette O "Cetylstearylalkohol", Henkel AG) | 3,00 |
| Paraffinöl dünnflüssig (E. Merck) | 3,00 |
| Isopropylmyristat | 1,50 |
| Abil AV 20 | 1,00 |
| Antioxidans enthaltend Butylhydroxytoluol "Oxynex 2004" (E. Merck) | 0,02 |
| Allantoin (E. Merck) | 0,30 |
| Sorbit "Karion F flüssig" (E. Merck) | 6,00 |
| Dinatriumsalz der Ethylendiamintetraessigsäure "Titriplex" (E. Merck) | 0,05 |
| Pantothenylalkohol | 0,30 |
| Konservierungsmittel (E. Merck) | 0,5 |
| demineralisiertes Wasser | ad 100 |

Beispiel B: Sonnenschutzmilch (O/W)

| | Gew. % |
|---|---|
| UV-Filter | q.s. |
| Mischung aus Cetyl-Dimethicone Copolyol, Polyglyceryl-4-Isostearat und Hexyllaurat "Abil WE 09" (Th. Goldschmidt) | 5,00 |
| Paraffinöl dünnflüssig (E. Merck) | 16,00 |
| Natriumchlorid (E. Merck) | 2,00 |
| Glycerin (E. Merck) | 3,00 |
| Konservierungsmittel (E. Merck) | q.s. |
| Wasser, demineralisiert | ad 100,00 |
| Parfümöl Delaila (Dragoco) | 0,50 |

Man mischt die Ingredienzien, wobei sie zum Homogenisieren gegebenenfalls auf 70-85 °C erhitzt werden; das Parfümöl wird bei 35-45 °C zugegeben.

Beispiel C: Sonnenschutzcreme (W/O)

| | | Gew. % |
|---|---|---|
| A | UV-Filter | q.s. |
| | POE Glycerol sorbitan oleostearat "Arlacel 581" (ICI) | 7,0 |
| | Paraffinöl dünnflüssig (E. Merck) | 6,0 |
| | PPG-15 Stearylether und Cyclomethicon 10 "Arlamol S 7" (ICI) | 2,0 |
| | 020 Kevit-Wachs "Lunacera M" (LW Fuller) | 5,0 |
| | Cyclomethicon "Dow Corning 344" (Dow Corning) | 4,00 |
| | Triglyceridöl "Miglyol 812" (Hüls Troisdorf AG) | 2,00 |
| B | Glycerin (Art.-Nr. 4093) (E. Merck) | 2,00 |
| | Magnesiumsulfat-Heptahydrat (E. Merck) | 0,17 |
| | Konservierungsmittel (E. Merck) | q.s. |
| | Wasser, demineralisiert | ad 100,00 |

Die Ingredienzien werden bei 70-85 °C homogenisiert; die Zubereitung wird gegebenenfalls bei 35-45 °C parfümiert.

Beispiel D: Sonnenschutzcreme (O/W)

| | | Gew. % |
|---|---|---|
| A | UV-Filter | q.s. |
| | Mischung aus Cetearylalkohol und Ceteareth-20 "Emulgade 1000 Ni" (Henkel) (2) | 10,00 |
| | Paraffinöl dickflüssig (E. Merck) (1) | 2,00 |
| | Dimethylsiloxan "Dow Corning 200 (100 cs)" (3) | 0,50 |
| | Oxynex 2004 (E. Merck) (1) | 0,10 |
| B | Glycerin (E. Merck) (1) | 5,00 |
| | Titriplex III (E. Merck) (1) | 0,10 |
| | Konservierungsmittel (E. Merck) (1) | q.s. |
| | Wasser, demineralisiert | ad 100,00 |

Die Ingredienzien werden bei 70 bis 85 °C homogenisiert. Die Zubereitung wird gegebenenfalls bei 35 bis 45 °C parfümiert.

Beispiel E: Sonnenschutzcreme (O/W)

Zur Neutralisierung von Eusolex® 232 wird das Tris(hydroxymethyl)aminomethan in Wasser gelöst und Eusolex® 232 unter Rühren zugegeben. Nach vollständiger Lösung werden die restlichen Ingredienzien bei 75-85 °C hinzugefügt und homogenisiert. Unter Rühren abkühlen und gegebenenfalls bei 40 °C parfümieren.

| | Gew. % |
|---|---|
| Verbindung aus Beispiel 1 | 5,00 |
| Stearinsäure (E. Merck) | 20,00 |
| Triglycerid-Öl "Miglyol 812" (Hüls Troisdorf AG) | 20,00 |
| UV-Filter 2-Phenylbenzimidazol-5-sulfonsäure "Eusolex 232" (E. Merck) | 1,50 |
| Tris (hydroxymethyl)aminomethan (E. Merck) | 0,66 |
| Sorbit "Karion F flüssig" (E. Merck) | 5,00 |
| Allantoin (E. Merck) (1) | 0,20 |
| Triethanolamin (E. Merck) (1) | 6,00 |
| Konservierungsmittel (E. Merck) (1) | q.s. |
| Wasser, demineralisiert | ad 100,00 |

Die Verbindung aus Beispiel 1 kann auch ersetzt werden durch ein Gemisch aus 3 % Verbindung aus Beispiel 1 und 2 % 3-(4-Methylbenzyliden)kampfer.

Beispiel F: Frisiergel (ölhaltig) mit UV-Filter

Die Verbindung aus Beispiel 1 kann auch durch ein Gemisch aus 2 % Verbindung aus Beispiel 1 und 1 % Benzophenon-3 bzw. 1 % 3-(4-Methylbenzyliden)kampfer ersetzt werden.

| | Gew. % |
|---|---|
| Verbindung aus Beispiel 1 | 3 |
| Polyoxyethylen-30-cetylstearylalkohol "Emulgin B 3" (Henkel AG) (2) | 13,00 |
| Polyolfettsäureester "Cetiol HE" (Henkel AG) (2) | 20,00 |
| Eutanol G "Octyldecanol" (Henkel AG) (2) | 5,00 |
| Konservierungsmittel (E. Merck) | q.s. |
| Wasser, demineralisiert | ad 100,00 |

Die Ingredienzien werden gemischt und bei 70 bis 85 °C homogenisiert. Die Zubereitung wird gegebenenfalls bei 35 bis 45 °C parfümiert.

Beispiel G: Haarkur mit UV-Filter (Creme O/W)

| | Gew. % |
|---|---|
| UV-Filter | 3,00 |
| Cetylstearylalkohol "Lanette O" (Henkel AG) | 2,50 |
| Polyoxyethylen-20-stearylalkohol "Emulgin B 2" (Henkel AG) | 1,00 |
| Wachsester (Jojobaöl-Substitut) "Cetiol J 600" (Henkel AG) | 1,00 |
| N-Cetyl-N,N,N-trimethylammoniumbromid (E. Merck) | 1,00 |
| Konservierungsmittel (E. Merck) | q.s. |
| Wasser, demineralisiert | ad 100,00 |

Die Herstellung erfolgt analog Beispiel A.

Die Verbindung aus Beispiel 1 kann auch ersetzt werden durch ein Gemisch aus der Verbindung aus Beispiel 1 (2 %) und Benzophenon-3 (1 %) bzw. 3-(4-Methylbenzyliden)kampfer (1 %).

Beispiel H: Sonnenöl

| | Gew. % |
|---|---|
| UV-Filter | q.s. |
| POE-40-Sorbitolseptaoleat "Arlatone T" (ICI) | 2,00 |
| Triglycerid Öl "Miglyol 812" (Hüls Troisdorf AG) (3) | 16,00 |
| Di-n-butyladipat "Cetiol B" (Henkel AG) (4) | 22,50 |
| Isopropylmyritat (Henkel AG) (4) | 7,50 |
| Paraffinöl dünnflüssig (E. Merck) | ad 100,00 |
| Antioxidans enthaltend Butylhydroxytoluol "Oxynex 2004" (E. Merck) | 0,2 |
| Konservierungsmittel | q.s. |
| Parfümöl 72979 (Haarmann & Reimer) | q.s. |

Die Herstellung erfolgt analog Beispiel A.

Beispiel I: Sonnenschutzgel (wässrig-alkoholisch)

Wässrige Phase:

| | Gew. % |
|---|---|
| Verbindung aus Beispiel 2 | 10,00 |
| Tris(hydroxymethyl)aminomethan (E. Merck) | 2,80 |
| Allantoin (E. Merck) | 0,20 |
| Sorbit "Karion F flüssig" (E. Merck) | 5,00 |
| Konservierungsmittel | q.s. |
| Wasser, demineralisiert | ad 100,00 |

Alkoholische Phase:

| | Gew. % |
|---|---|
| Parfümöl (Haarmann & Reimer) | 0,30 |
| POE-35 hydriertes Castor Öl "Arlatone 980" (ICI) | 1,00 |
| Carboxyvinyl-Polymeres "Carbopol 940" (Goodrich) | 1,50 |
| Wasser, demineralisiert | 35.50 |
| Triethanolamin (E. Merck) | 3,00 |
| Wasser, demineralisiert | 10,00 |
| Ethanol (96%ig) (E. Merck) | ad 100,00 |

Zur Neutralisierung des UV-Filters aus Beispiel 2 wird das Tris(hydroxymethyl)aminomethan in Wasser gelöst und der UV-Filter aus Beispiel 2 unter Rühren zugegeben. Nach vollständiger Lösung werden die restlichen Rohstoffe zugegeben und zum Lösung auf 75 °C erhitzt. Anschließend wird unter Rühren abgekühlt.

Beispiel J: Sonnenschutzcreme (O/W)

| | | % |
|---|---|---|
| A | UV-Filter<br>Stearinsäure (E. Merck)<br>Triglycerid-Öl "Miglyol 812" | q.s.<br>20,00<br>20,00 |
| B | Verbindung aus Beispiel 2 (E. Merck) (1)<br>Tris(hydroxymethyl)aminomethan (E. Merck) (1)<br>Sorbit "Karion F flüssig" (E. Merck) (1)<br>Allantoin (E. Merck) (1)<br>Triethanolamin (E. Merck) (1)<br>Konservierungsmittel (E. Merck) (1)<br>Wasser, demineralisiert | 10,00<br>2,80<br>5,00<br>0,20<br>6,00<br>q.s.<br>ad 100,00 |

Als UV-Filter können wahlweise eingesetzt werden:

- 3 % 3-(4-Methylbenzyliden)kampfer oder
- 5 % Octyldimethylpaba oder
- 2 % Benzophenon-3 oder
- 4 % Octylmethoxycinnamat

Herstellung:

Zur Neutralisierung des UV-Filters aus Beispiel 2 wird das Tris(hydroxymethyl)aminomethan im Wasser der Phase B gelöst und der UV-Filter aus Beispiel 4 unter Rühren zugegeben.

Nach vollständiger Lösung werden die restlichen Rohstoffe der Phase B zugegeben und auf 80 °C erhitzt. Die Phase A wird auf 75 °C erhitzt. Die Phase B wird langsam in Phase A eingerührt. Homogenisieren. Unter Rühren abkühlen und gegebenenfalls bei 40 °C parfümieren.

Beispiel K: Sonnenschutzcreme (O/W)

| | | | | % |
|---|---|---|---|---|
| A | UV-Filter | | | q.s. |
| | Stearinsäure (E. Merck) | | | 20,00 |
| | Triglycerid-Öl "Miglyol 812" (Hüls Troisdorf) | | | 20,00 |
| B | Verbindung aus Beispiel 2 (E. Merck) | (1) | | 5,00 |
| | Natriumhydroxid (10%ige Lösung) (E. Merck) | (1) | | 0,40 |
| | Sorbit "Karion F flüssig" (E. Merck) | (1) | | 5,00 |
| | Allantoin (E. Merck) | (1) | | 0,20 |
| | Triethanolamin (E. Merck) | (1) | | 6,00 |
| | Konservierungsmittel | (1) | | q.s. |
| | Wasser, demineralisiert | | | ad 100,00 |

Als UV-Filter können wahlweise eingesetzt werden:

- 3 % 3-(4-Methylbenzyliden)kampfer oder
- 5 % Octyldimethylpaba oder
- 2 % Benzophenon-3 oder
- 4 % Octylmethoxycinnamat

Herstellung:

Der UV-Filter aus Beispiel 2 wird zur Neutralisierung in die Natriumhydroxidlösung und das Wasser der Phase B eingerührt. Nach vollständiger Lösung werden die restlichen Rohstoffe der Phase B zugegeben und auf 80 °C erhitzt. Die Phase A wird auf 75 °C erhitzt. Die Phase B wird langsam in Phase A eingerührt. Homogenisieren. Unter Rühren abkühlen und gegebenenfalls bei 40 °C parfümieren.

Beispiel L: Sonnenschutzcreme (O/W)

| | | | % |
|---|---|---|---|
| A | UV-Filter | | q.s. |
| | Stearinsäure (E. Merck) | (1) | 20,00 |
| | Triglycerid-Öl "Miglyol 812" (Hüls Troisdorf AG) | (2) | 20,00 |
| B | UV-Filter aus Beispiel 2 (E. Merck) | (1) | 5,00 |
| | Sorbit "Karion F flüssig" (E. Merck) | (1) | 5,00 |
| | Allantoin (E. Merck) | (1) | 0,20 |
| | Triethanolamin (E. Merck) | (1) | 6,17 |
| | Konservierungsmittel (E. Merck) | (1) | q.s. |
| | Wasser, demineralisiert | | ad 100,00 |

Als UV-Filter können eingesetzt werden:

- 3 % 3-(4-methylbenzyliden)kampfer oder
- 5 % Octyldimethylpaba oder
- 2 % Benzophenon-3 oder
- 4 % Octylmethoxycinnamat

Herstellung:

Zur Neutralisierung des UV-Filters aus Beispiel 2 wird das Triethanolamin im Wasser der Phase B gelöst und der UV-Filter aus Beispiel 4 unter Rühren zugegeben. Nach vollständiger Lösung werden die restlichen Rohstoffe der Phase B zugegeben und auf 80 °C erhitzt. Die Phase A wird auf 75 °C erhitzt. Die Phase B wird langsam in Phase A eingerührt. Homogenisieren. Unter Rühren abkühlen und gegebenenfalls bei 40 °C parfümieren.

**Patentansprüche**

**Patentansprüche für folgende Vertragsstaaten : AT, BE, CH, DE, FR, GB, IT, LI, NL, SE**

**1.** Dialkoxybenzylidenkampfer-Derivate der Formel I

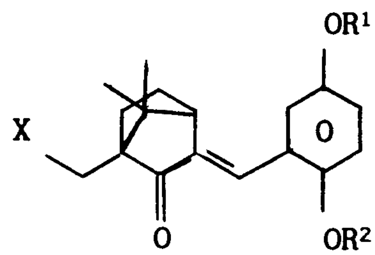

I

worin

| | |
|---|---|
| $R^1$ und $R^2$ | jeweils unabhängig voneinander einen geradkettigen oder verzweigten Alkylrest mit 1 bis 10 C-Atomen, und |
| X | H oder $SO_3H$ |

bedeuten.

**2.** Verbindungen nach Anspruch 1, ausgewählt aus der Gruppe bestehend aus
3-(2',5'-Dimethoxybenzyliden)kampfer
3-(2',5'-Diethoxybenzyliden)kampfer
3-(2',5'-Dipropoxybenzyliden)kampfer.

**3.** Verbindungen nach Anspruch 1, ausgewählt aus der Gruppe bestehend aus
3-(2',5'-Dimethoxybenzyliden)kampfer-10-sulfonsäure
3-(2',5'-Diethoxybenzyliden)kampfer-10-sulfonsäure
3-(2',5'-Dipropoxybenzyliden)kampfer-10-sulfonsäure.

**4.** Verfahren zur Herstellung der Verbindungen der Formel I nach einem der Ansprüche 1 bis 3, dadurch gekennzeichnet, daß man einen aromatischen Aldehyd der Formel II,

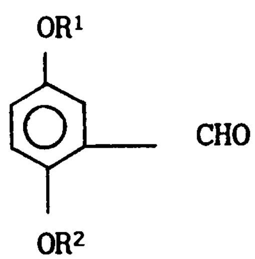

II

worin $R^1$ und $R^2$ die in Anspruch 1 angegebene Bedeutung besitzen mit Kampfer oder Kamper-10-sulfonsäure in Gegenwart einer mineralischen Base oder eines Dialkylacyloxyborans kondensiert.

**5.** Kosmetische Zubereitung, dadurch gekennzeichnet, daß sie eine wirksame Menge mindestens einer Verbindung der Formel I nach Anspruch 1 in einem kosmetisch verträglichen Träger enthält.

**6.** Kosmetische Zubereitung nach Anspruch 5, wobei der Träger mindestens eine Fettphase aufweist, dadurch gekennzeichnet, daß sie mindestens eine Verbindung der Formel I, worin X H bedeutet, enthält.

**7.** Kosmetische Zubereitung nach Anspruch 5, wobei der Träger mindestens eine wäßrige Phase aufweist, dadurch gekennzeichnet, daß sie mindestens eine Verbindung der Formel I, worin X $SO_3H$ bedeutet,

enthält.

8. Kosmetische Zubereitung nach einem der Ansprüche 5 bis 7, dadurch gekennzeichnet, daß es 1 bis 10 Gew.% mindestens einer Verbindung der Formel I enthält.

9. Kosmetische Zubereitung nach einem der Ansprüche 5 bis 8, dadurch gekennzeichnet, daß sie zusätzlich einen UV-B-Filter enthält.

10. Verwendung der Verbindungen der Formel I nach einem der Ansprüche 1 bis 3 als kosmetisches Produkt.

11. Verbindung der Formel I nach einem der Ansprüche 1 bis 3 zur Verwendung als Arzneimittel.

12. Verbindung der Formel I zur Verwendung bei der vorbeugenden Behandlung von Entzündungen und Allergien der Haut.

13. Verbindung der Formel I zur Verwendung in der Prevention bestimmter Krebsarten.

14. Pharmazeutische Zubereitung, dadurch gekennzeichnet, daß sie eine wirksame Menge mindestens einer Verbindung der Formel I nach einem der Ansprüche 1 bis 3 in einem physiologisch unbedenklichen Träger oder Exzipienten enthält.

15. Pharmazeutische Zubereitung nach Anspruch 15, dadurch gekennzeichnet, daß sie topisch angewandt wird.

16. Verwendung der Verbindungen der Formel I nach einem der Ansprüche 1 bis 3 zur Herstellung von Arzneimitteln.

**Patentansprüche für folgenden Vertragsstaat : ES**

1. Verfahren zur Herstellung der Dialkoxybenzylidenkampfer-Derivate der Formel I

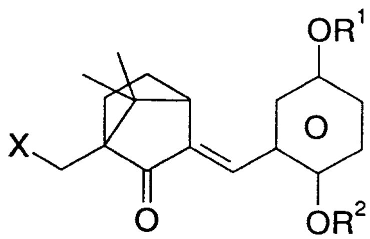

I

worin

$R^1$ und $R^2$ jeweils unabhängig voneinander einen geradkettigen oder verzweigten Alkylrest mit 1 bis 10 C-Atomen, und

X H oder $SO_3H$

bedeuten,

dadurch gekennzeichnet, daß man einen aromatischen Aldehyd der Formel II,

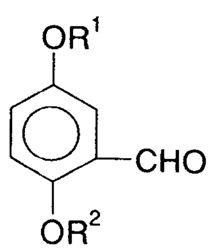

II

worin $R^1$ und $R^2$ die angegebene Bedeutung besitzen mit Kampfer oder Kampfer-10-sulfonsäure in Gegenwart einer mineralischen Base oder eines Dialkylacyloxyborans kondensiert.

2. Verfahren nach Anspruch 1 zur Herstellung der Verbindungen, ausgewählt aus der Gruppe besteht aus
3-(2',5'-Dimethoxybenzyliden)kampfer
3-(2',5'-Diethoxybenzyliden)kampfer
3-(2',5'-Dipropoxybenzyliden)kampfer.

3. Verfahren nach Anspruch 1 zur Herstellung der Verbindungen, ausgewählt aus der Gruppe bestehend aus
3-(2',5'-Dimethoxybenzyliden)kampfer-10-sulfonsäure
3-(2',5'-Diethoxybenzyliden)kampfer-10-sulfonsäure
3-(2',5'-Dipropoxybenzyliden)kampfer-10-sulfonsäure.

4. Kosmetische Zubereitung, dadurch gekennzeichnet, daß sie eine wirksame Menge mindestens einer Verbindung der Formel I nach Anspruch I in einem kosmetisch verträglichen Träger enthält.

5. Kosmetische Zubereitung nach Anspruch 4, wobei der Träger mindestens eine Fettphase aufweist, dadurch gekennzeichnet, daß sie mindestens eine Verbindung der Formel I, worin X H bedeutet, enthält.

6. Kosmetische Zubereitung nach Anspruch 4, wobei der Träger mindestens eine wäßrige Phase aufweist, dadurch gekennzeichnet, daß sie mindestens eine Verbindung der Formel I, worin X $SO_3H$ bedeutet, enthält.

7. Kosmetische Zubereitung nach einem der Ansprüche 4 bis 6, dadurch gekennzeichnet, daß es 1 bis 10 Gew.% mindestens einer Verbindung der Formel I enthält.

8. Kosmetische Zubereitung nach einem der Ansprüche 4 bis 7, dadurch gekennzeichnet, daß sie zusätzlich einen UV-B-Filter enthält.

9. Verwendung der Verbindungen der Formel I nach einem der Ansprüche 1 bis 3 als kosmetisches Produkt.

**Claims**

**Claims for the following Contracting States : AT, BE, CH, DE, FR, GB, IT, LI, NL, SE**

1. Dialkoxybenzylidenecamphor derivatives of the formula I

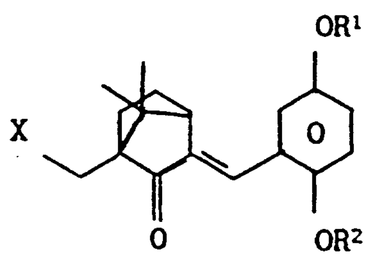

I

in which

$R^1$ and $R^2$, in each case independently of one another, are a straight-chain or branched alkyl radical having 1 to 10 C atoms, and
X is H or $SO_3H$.

2. Compounds according to Claim 1, selected from the group consisting of
3-(2',5'-dimethoxybenzylidene)camphor
3-(2',5'-diethoxybenzylidene)camphor
3-(2',5'-dipropoxybenzylidene)camphor.

**3.** Compounds according to Claim 1, selected from the group consisting of
3-(2',5'-dimethoxybenzylidene)camphor-10-sulphonic acid
3-(2',5'-diethoxybenzylidene)camphor-10-sulphonic acid
3-(2',5'-dipropoxybenzylidene)camphor-10-sulphonic acid.

**4.** Process for the preparation of the compounds of the formula I according to one of Claims 1 to 3, characterised in that an aromatic aldehyde of the formula II

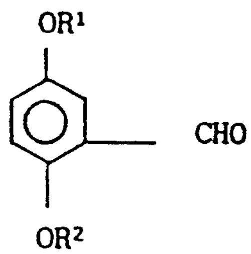

II

in which $R^1$ and $R^2$ have the meaning indicated in Claim 1, is condensed with camphor or camphor-10-sulphonic acid in the presence of an inorganic base or a dialkylacyloxyborane.

**5.** Cosmetic preparation, characterised in that it contains an effective amount of at least one compound of the formula I according to Claim 1 in a cosmetically tolerable vehicle.

**6.** Cosmetic preparation according to Claim 5, in which the vehicle has at least one lipid phase, characterised in that it contains at least one compound of the formula I in which X is H.

**7.** Cosmetic preparation according to Claim 5, in which the vehicle has at least one aqueous phase, characterised in that it contains at least one compound of the formula I in which X is $SO_3H$.

**8.** Cosmetic preparation according to one of Claims 5 to 7, characterised in that it contains 1 to 10 % by weight of at least one compound of the formula I.

**9.** Cosmetic preparation according to one of Claims 5 to 8, characterised in that it additionally contains a UV-B filter.

**10.** Use of the compounds of the formula I according to one of Claims 1 to 3 as a cosmetic product.

**11.** Compound of the formula I according to one of Claims 1 to 3 for use as a medicament.

**12.** Compound of the formula I for use in the prophylactic treatment of inflammations and allergies of the skin.

**13.** Compound of the formula I for use in the prevention of certain types of cancer.

**14.** Pharmaceutical preparation, characterised in that it contains an effective amount of at least one compound of the formula I according to one of Claims 1 to 3 in a physiologically acceptable vehicle or excipient.

**15.** Pharmaceutical preparation according to Claim 15, characterised in that it is used topically.

**16.** Use of the compounds of the formula I according to one of Claims 1 to 3 for the preparation of medicaments.

**Claims for the following Contracting State : ES**

1. Process for the preparation of dialkoxybenzylidenecamphor derivatives of the formula I

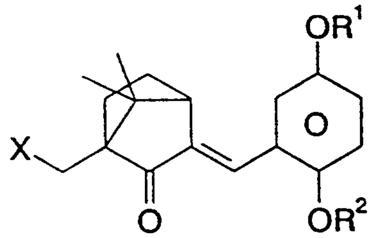

I

in which

$R^1$ and $R^2$, in each case independently of one another, are a straight-chain or branched alkyl radical having 1 to 10 C atoms, and

X is H or $SO_3H$ characterised in that an aromatic aldehyde of the formula II

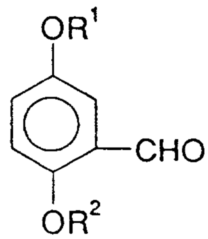

II

in which $R^1$ and $R^2$ have the meaning indicated, is condensed with camphor or camphor-10-sulphonic acid in the presence of an inorganic base or a dialkylacyloxyborane.

2. Process according to Claim 1 for the preparation of compounds, selected from the group consisting of
3-(2',5'-dimethoxybenzylidene)camphor
3-(2',5'-diethoxybenzylidene)camphor
3-(2',5'-dipropoxybenzylidene)camphor.

3. Process according to Claim 1 for the preparation of compounds, selected from the group consisting of
3-(2',5'-dimethoxybenzylidene)camphor-10-sulphonic acid
3-(2',5'-diethoxybenzylidene)camphor-10-sulphonic acid
3-(2',5'-dipropoxybenzylidene)camphor-10-sulphonic acid.

4. Cosmetic preparation, characterised in that it contains an effective amount of at least one compound of the formula I according to Claim 1 in a cosmetically tolerable vehicle.

5. Cosmetic preparation according to Claim 4, in which the vehicle has at least one lipid phase, characterised in that it contains at least one compound of the formula I in which X is H.

6. Cosmetic preparation according to Claim 4, in which the vehicle has at least one aqueous phase, characterised in that it contains at least one compound of the formula I in which X is $SO_3H$.

7. Cosmetic preparation according to one of Claims 4 to 6, characterised in that it contains 1 to 10 % by weight of at least one compound of the formula I.

8. Cosmetic preparation according to one of Claims 4 to 7, characterised in that it additionally contains a UV-B filter.

9. Use of the compounds of the formula I according to one of Claims 1 to 3 as a cosmetic product.

**Revendications**

**Revendications pour les Etats contractants suivants : AT, BE, CH, DE, FR, GB, IT, LI, NL, SE**

1. Dérivé de dialcoxybenzylidènecamphre de formule I

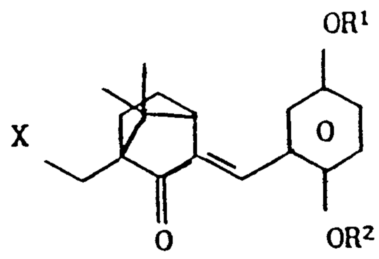

. I

dans laquelle

| | |
|---|---|
| $R^1$ et $R^2$ | désignent respectivement, indépendamment l'un de l'autre, un radical alkyle à chaîne droite ou ramifiée avec 1 à 10 atomes C et |
| X | désigne H ou $SO_3H$. |

2. Composes selon la revendication 1, choisis parmi le groupe constitué des
3-(2',5'-diméthoxybenzylidène)-camphre
3-(2',5'-diéthoxybenzylidène)-camphre
3-(2',5'-dipropoxybenzylidène)-camphre

3. Composes selon la revendication 1, choisis parmi le groupe constitué de
l'acide3-(2',5'-diméthoxybenzylidène)-campho-10-sulfonique
l'acide 3-(2',5'-diéthoxybenzylidène)-campho-10-sulfonique
l'acide3-(2',5'-dipropoxybenzylidène)-campho-10-sulfonique

4. Procédé de préparation des composés de formule I selon l'une des revendications 1 à 3, caractérisé en ce qu'on effectue la condensation d'un aldéhyde aromatique de formule II

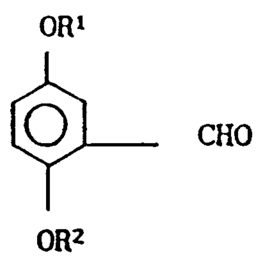

II

dans laquelle $R^1$ et $R^2$ possèdent la signification indiquée dans la revendication 1 avec du camphre ou de l'acide campho-10-sulfonique en présence d'une base minérale ou d'un dialkylacyloxyborane.

5. Préparation cosmétique, caractérisée en ce qu'elle contient une quantité active d'au moins un composé de formule I selon la revendication 1 dans un support compatible cosmétiquement.

6. Préparation cosmétique selon la revendication 5, le support présentant au moins une phase grasse, caractérisée en ce qu'elle contient au moins un composé de formule I, dans laquelle X désigne H.

7. Préparation cosmétique selon la revendication 5, dans laquelle le support présente au moins une phase aqueuse, caractérisée en ce qu'elle contient au moins un composé de formule I, dans laquelle X désigne $SO_3H$.

**8.** Préparation cosmétique selon l'une des revendications 5 à 7, caractérisée en ce qu'elle contient au moins 1 à 10 % en poids d'au moins un composé de formule I.

**9.** Préparation cosmétique selon l'une des revendications 5 à 8, caractérisée en ce qu'elle contient par ailleurs un filtre UV-B.

**10.** Mise en oeuvre des composés de formule I selon l'une des revendications 1 à 3 comme produit cosmétique.

**11.** Composé de formule I selon l'une des revendications 1 à 3 à mettre en oeuvre à titre de médicament.

**12.** Composé de formule I à mettre en oeuvre dans le traitement prophylactique d'inflammations et allergies de la peau.

**13.** Composé de formule I à mettre en oeuvre pour la prévention de certaines formes de cancers.

**14.** Préparation pharmaceutique, caractérisée en ce qu'elle contient une quantité active d'au moins un composé de formule I selon l'une des revendications 1 à 3 dans un support ou excipient physiologiquement neutre.

**15.** Préparation pharmaceutique selon la revendication 15, caractérisée en ce qu'elle est appliquée localement.

**16.** Mise en oeuvre des composés de formule I selon l'une des revendications 1 à 3 en vue de la fabrication de médicaments.

**Revendications pour l'Etat contractant suivant : ES**

**1.** Procédé de préparation d'un dérivé de dialcoxybenzylidènecamphre de formule I

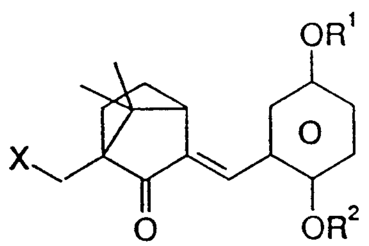

I

dans laquelle

- $R^1$ et $R^2$ désignent respectivement, indépendamment l'un de l'autre, un radical alkyle à chaîne droite ou ramifiée avec 1 à 10 atomes C et
- X désigne H ou $SO_3H$,

caractérisé en ce qu'on effectue la condensation d'un aldéhyde aromatique de formule II

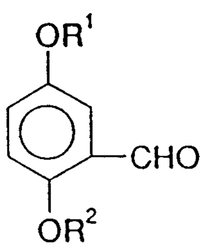

II

dans laquelle $R^1$ et $R^2$ possèdent la signification indiquée ci-dessus avec du camphre ou de l'acide campho-10-sulfonique en présence d'une base minérale ou d'un dialkylacyloxyborane.

2. Procédé selon la revendication 1 pour la préparation de composés choisis parmi le groupe constitué des
3-(2',5'-diméthoxybenzylidène)-camphre
3-(2',5'-diéthoxybenzylidène)-camphre
3-(2',5'-dipropoxybenzylidène)-camphre

3. Procédé selon la revendication 1 pour la préparation de composés choisis parmi le groupe constitué de
l'acide3-(2',5'-diméthoxybenzylidène)-campho-10-sulfonique
l'acide 3-(2',5'-diéthoxybenzylidène)-campho-10-sulfonique
l'acide3-(2',5'-dipropoxybenzylidène)-campho-10-sulfonique

4. Préparation cosmétique, caractérisée en ce qu'elle contient une quantité active d'au moins un composé de formule I selon la revendication 1 dans un support compatible cosmétiquement.

5. Préparation cosmétique selon la revendication 4, le support présentant au moins une phase grasse, caractérisée en ce qu'elle contient au moins un composé de formule I, dans laquelle X désigne H.

6. Préparation cosmétique selon la revendication 4, dans laquelle le support présente au moins une phase aqueuse, caractérisée en ce qu'elle contient au moins un composé de formule I, dans laquelle X désigne $SO_3H$.

7. Préparation cosmétique selon l'une des revendications 4 à 6, caractérisée en ce qu'elle contient au moins 1 à 10 % en poids d'au moins un composé de formule I.

8. Préparation cosmétique selon l'une des revendications 4 à 7, caractérisée en ce qu'elle contient par ailleurs un filtre UV-B.

9. Mise en oeuvre des composés de formule I selon l'une des revendications 1 à 3 comme produit cosmétique.